# EUROPÄISCHE PATENTANMELDUNG

(11) **EP 1 207 148 A1**
(43) Veröffentlichungstag der Anmeldung: **22.05.2002**
(21) Anmeldenummer: 01126255.7
(22) Anmeldetag: 06.11.2001
(51) Int. Cl.: C07C 51/10, C07C 57/32, C07C 57/58

(54) **Verfahren zur Herstellung von Phenylessigsäurederivaten**

(30) Priorität: 18.11.2000 DE 10057262
(71) Anmelder: Clariant GmbH, 65929 Frankfurt am Main (DE)
(72) Erfinder: Geissler, Holger, Dr., 55116 Mainz (DE)

(57) **Zusammenfassung**

Verfahren zur Herstellung von Phenylessigsäurederivaten der allgemeinen Formel (I) durch Umsetzung von Benzylchloriden der allgemeinen Formel (II) 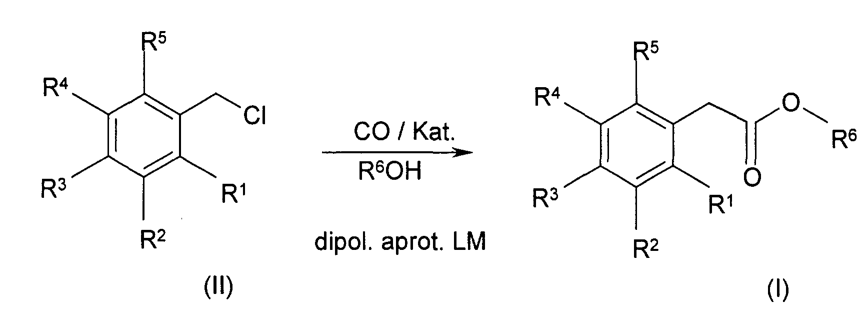 mit einer Verbindung der allgemeinen Formel R⁶OH und mit Kohlenmonoxid in einem dipolar aprotischen Lösungsmittel in Gegenwart eines Katalysators, der mindestens eine Verbindung eines Übergangsmetalls der VIII. Nebengruppe des Periodensystems enthält,
wobei R¹, R², R³, R⁴, und R⁵ unabhängig voneinander für folgende Reste stehen: ein Wasserstoff oder Fluoratom;
ein CH₂CI-Rest;
eine HO₂CCH=CH-, NC- oder CF₃-Gruppe;
einen Alkyl-, Alkoxy-, oder Acyloxyrest, mit jeweils 1 bis 18 Kohlenstoffatomen; oder einen C₆-C₁₈-Aryloxy, Aryl- oder Heteroarylrest, wobei als Heteroatome 1 bis 3 Atome der Gruppe O, N und/oder S vorhanden sind;
einen R⁸₂P(=O)-, R⁹C(=O)-, R⁹OC(=O)-, R⁹OC(=O)CH=CH-, R¹⁰C(=O)-, R¹⁰OC(=O)-, R¹⁰OC(=O)CH=CH-, oder R¹⁰₂P(=O)-Rest, worin R⁸ einen C₁-C₄-Alkylrest, R⁹ einen C₁-C₁₈-Alkylrest oder Wasserstoff und R¹⁰ einen C₆-C₁₈-Arylrest darstellen;
oder worin mindestens zwei der Reste R¹, R², R³, R⁴, und R⁵ untereinander verknüpft sind und mindestens einen aliphatischen oder aromatischen Ring mit 5 bis 18 C-Atomen bilden;
R⁶ steht für Wasserstoff oder einen C₁-C₁₈-Alkyl- oder C₆-C₁₈-Arylrest.

## Beschreibung

Die vorliegende Patentanmeldung beschreibt ein Verfahren zur Herstellung von substituierten Phenylessigsäuren oder deren Estern durch Umsetzung von substituierten Benzylchloriden mit Kohlenmonoxid und Alkoholen oder Wasser in Gegenwart eines Katalysators und eines dipolar aprotischen Lösemittels.

Substituierte Phenylessigsäuren und deren Ester sind wichtige Intermediate in der pharmazeutischen sowie der agrochemischen Industrie.

Die Synthese von Phenylessigsäuren ausgehend von Benzylchloriden sowie die Synthese von Phenylessigsäureestern wird von Colquhoun, Thompson und Twigg beschrieben ("Carbonylation, Direct Synthesis of Carbonyl Compounds" Plenum Press, New York, 1991, Seiten 91 - 97 und 111 - 119). Die Umsetzungen der Benzylchloride mit Kohlenmonoxid und Wasser oder einem Alkohol gelingt danach in Gegenwart katalytischer Mengen von Nickel-, Cobalt-, Eisen-, Ruthenium-, Rhodium-, oder Palladiumverbindungen und unter Zusatz stöchiometrischer Mengen einer Base um den in der Reaktion erzeugten Halogenwasserstoff zu neutralisieren.

Aufgrund der Nachteile der bekannten Verfahren und des steigenden Bedarfs bestand die Aufgabe ein Verfahren zu finden, dass es erlaubt substituierte oder unsubstituierte Benzylchloride auf einfache und wirtschaftliche Weise und in hohen Ausbeuten zu den korrespondierenden Phenylessigsäurederivaten umzusetzen.

Diese Aufgabe wird gelöst durch ein Verfahren zur Herstellung von Phenylessigsäurederivaten der allgemeinen Formel (I) durch Umsetzung von Benzylchloriden der allgemeinen Formel (II) 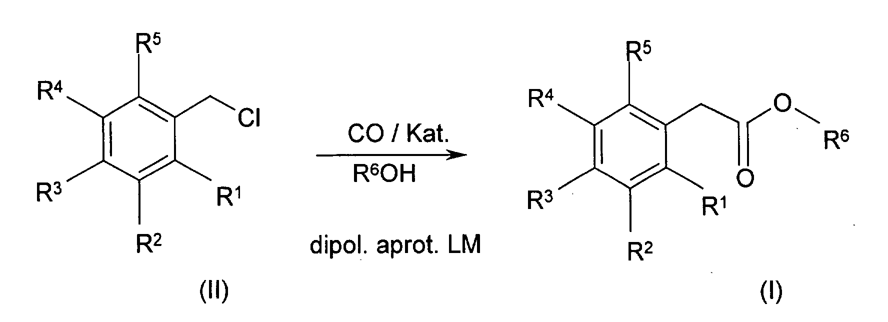 mit einer Verbindung der allgemeinen Formel R⁶OH und mit Kohlenmonoxid, in einem dipolar aprotischen Lösungsmittel in Gegenwart eines Katalysators, der mindestens eine Verbindung eines Übergangsmetalls der VIII. Nebengruppe des Periodensystems enthält,
wobei R¹, R², R³, R⁴, und R⁵ unabhängig voneinander für folgende Reste stehen:
ein Wasserstoff oder Fluoratom;
ein CH₂Cl-Rest;
eine HO₂CCH=CH-, NC- oder CF₃-Gruppe;
einen Alkyl-, Alkoxy-, oder Acyloxyrest, mit jeweils 1 bis 18 Kohlenstoffatomen; oder einen C₆-C₁₈-Aryloxy, Aryl- oder Heteroarylrest, wobei als Heteroatome 1 bis 3 Atome der Gruppe O, N und/oder S vorhanden sind;
einen R⁸₂P(=O)-, R⁹C(=O)-, R⁹OC(=O)-, R⁹OC(=O)CH=CH-, R¹⁰C(=O)-, R¹⁰OC(=O)-, R¹⁰OC(=O)CH=CH-, oder R¹⁰₂P(=O)-Rest, worin R⁸ einen C₁-C₄-Alkylrest, R⁹ einen C₁-C₁₈-Alkylrest oder Wasserstoff und R¹⁰ einen C₆-C₁₈-Arylrest darstellen;
oder worin mindestens zwei der Reste R¹, R², R³, R⁴, und R⁵ untereinander verknüpft sind und mindestens einen aliphatischen oder aromatischen Ring mit 5 bis 18 C-Atomen bilden.

R⁶ steht für Wasserstoff, einen C₁-C₁₈-Alkyl-, insbesondere einen C₁-C₄-Alkyl-, oder C₆-C₁₈-Arylrest.

Die Reste R¹, R², R³, R⁴, und R⁵ bedeuten insbesondere unabhängig voneinander Wasserstoff, Fluor, C₁-C₈-Alkyl, oder C₁-C₈-Alkoxy oder zwei der Reste R¹, R², R³, R⁴, und R⁵ sind untereinander verknüpft und bilden vorzugsweise einen aliphatischen oder aromatischen Ring mit 5 bis 10 C-Atomen. Vorzugsweise bedeuten die Reste R¹, R², R³, R⁴, und R⁵ unabhängig voneinander Wasserstoff, Fluor und C₁-C₄-Alkyl.

Das erfindungsgemäße Verfahren kann in Anwesenheit oder Abwesenheit eines ionischen Halogenids durchgeführt werden.

Üblicherweise ist das ionische Halogenid ein Alkali-, Ammonium-, Alkylammonium- oder Phosphoniumhalogenid, insbesondere ein Alkali- oder Ammoniumhalogenid, wobei Halogenid die Bedeutung Chlorid, Bromid oder Jodid, insbesondere Chlorid oder Bromid, bevorzugt Chlorid hat.

Man kann als ionisches Halogenid Ammoniumbromid, Lithiumbromid, Natriumbromid, Kaliumbromid, Tetrabutylphosphoniumbromid, Ammoniumchlorid, Dimethylammoniumchlorid, Diethanolammoniumchlorid, Lithiumchlorid, Natriumchlorid, Kaliumchlorid, Tetrabutylphosphoniumchlorid, Ammoniumjodid, Lithiumjodid, Natriumjodid, Kaliumjodid und/oder Tetrabutylphosphoniumjodid, insbesondere Lithiumchlorid, Ammoniumchlorid, Dimethylammoniumchlorid, und/oder Diethanolammoniumchlorid einsetzen.

Bei dem erfindungsgemäßen Verfahren kann aber auch auf die Anwesenheit des ionischen Halogenids verzichtet werden und das Verfahren in Abwesenheit des ionischen Halogenids durchgeführt werden.
In einer besonderen Ausführungsform des Verfahrens kann das ionische Halogenid insbesondere wenn der Katalysator Palladium enthält eingesetzt werden.

Der Katalysator enthält mindestens eine Übergangsmetallverbindung der VIII-Nebengruppe, insbesondere Palladium-, Nickel- und/oder Kobaltverbindungen, bevorzugt Palladium- und/oder Kobaltverbindungen, besonders bevorzugt Palladiumverbindungen.
Man kann für das erfindungsgemäße Verfahren auch einen Palladiumkatalysator, der auf einem Trägermaterial aufgebrachtes Nickel, Kobalt oder Palladium enthält, einsetzen. Ein derartiger Träger-Katalysator hat den Vorzug, dass er sich aus dem Reaktionsgemisch, beispielsweise durch Filtration, auf einfache Weise abtrennen lässt.

Bei der Verwendung von Palladiumkatalysatoren hat es sich in einer Reihe von Fällen bewährt, dass der Palladiumkatalysator mindestens eine Palladium-(II)-Verbindung, insbesondere PdCl₂, PdBr₂ oder Pd(OAc)₂ bevorzugt PdCl₂, oder mindestens eine Palladium-(0)-Verbindung, insbesondere Pd₂dba₃, worin dba für Dibenzylidenaceton steht, Pd(P(C₆H₅)₃)₄ oder Pd(C₈H₁₂)₂, bevorzugt Pd₂dba₃ enthält.

In einer Reihe von Fällen hat es sich ferner als günstig erwiesen, dass der Katalysator zusätzlich einen Liganden, insbesondere eine Phosphinverbindung, enthält.

Als Phosphinverbindung kommt beispielsweise ein Monophosphin, insbesondere ein Tri-(C₁-C₆)-alkylphosphin oder ein Triarylphosphin, oder ein Diphosphin in Betracht. Mit gutem Erfolg kann man Triphenylphosphin, Tritolylphosphin, Bis(diphenylphosphino)ethan, 1,3-Bis(diphenylphosphino)propan oder 1,4-Bis(diphenylphosphino)butan, insbesondere Triphenylphosphin einsetzen.
Nach einer besonderen Ausführungsform enthält der Palladiumkatalysator eine Bis(triphenylphosphin)-palladium(II)-Verbindung, beispielsweise Bis(triphenylphosphin)-palladium(II)-chlorid oder Bis(triphenylphosphin)-palladium(II)-bromid.

Man setzt den Katalysator üblicherweise in einer Menge von 0,00001 bis 0,3 Mol Übergangsmetall, insbesondere 0,0001 bis 0,2 Mol Übergangsmetall, bevorzugt 0,0005 bis 0,1 Mol Übergangsmetall je Mol Benzylchlorid der Formel (II) ein.

In einer Vielzahl von Fällen genügt es, die Umsetzung bei einem CO-Druck von 0,5 bis 20 MPa, insbesondere 0,8 bis 10, bevorzugt 1,0 bis 6 MPa durchzuführen.

Üblicherweise kann man die Umsetzung bei einer Temperatur von 20 bis 220°C, insbesondere bei 40 bis 200°C, bevorzugt bei 60 bis 180°C mit gutem Erfolg durchgeführt.

Als dipolar aprotisches Lösungsmittel eignet sich Dioxan, Tetrahydrofuran, N-(C₁-C₁₈-Alkyl)pyrrolidon, Ethylenglykoldimethylether, C₁-C₄-Alkylester aliphatischer C₁-C₆-Carbonsäuren, C₁-C₆-Dialkylether, N,N-Di-(C₁-C₄-alkyl)amide aliphatischer C₁-C₄-Carbonsäuren, Sulfolan, 1,3-Di-(C₁-C₈-alkyl)-2-imidazolidinon, N-(C₁-C₈-Alkyl)caprolactam, N,N,N',N'-Tetra-(C₁-C₈-alkyl)harnstoff, 1,3-Di-(C₁-C₈-alkyl)-3,4,5,6-tetrahydro-2(1 H)-pyrimidon, N,N,N',N'-Tetra-(C₁-C₈-alkyl)sulfamid, 4-Formylmorpholin, 1-Formylpiperidin oder 1-Formylpyrrolidin, insbesondere N-(C₁-C₁₈-Alkyl)pyrrolidon, N,N-Di-(C₁-C₄-Alkyl)amide aliphatischer C₁-C₄-Carbonsäuren, 4-Formylmorpholin, 1-Formylpiperidin oder 1-Formylpyrrolidin, bevorzugt N-Methylpyrrolidon, N-Octylpyrrolidon, N-Dodecylpyrrolidon, N,N-Dimethylformamid, N,N-Dimethylacetamid, 4-Formylmorpholin, 1-Formylpiperidin oder 1-Formylpyrrolidin, besonders bevorzugt N-Methylpyrrolidon, N,N-Dimethylformamid oder N,N-Dimethylacetamid. Es lassen sich auch Mischungen der vorstehend genannten dipolar aprotischen Lösungsmittel verwenden.

In der ganz besonders bevorzugten Variante wird N-Methylpyrrolidon als Lösemittel eingesetzt.
Beim Einsatz von N-Methylpyrrolidon als Lösemittel kann der entstehende Chlorwasserstoff im Anschluss an die Reaktion als wässriger Chlorwasserstoff isoliert und das Lösemittel N-Methylpyrrolidon wieder eingesetzt werden.

Man kann die Verbindung der Formel R⁶OH, entsprechend einem Alkylalkohol oder Wasser, in einer Menge von je 0,1 bis 50 Mol je Mol Benzylchlorid der Formel (II) zusetzen. Üblicherweise führt man die Umsetzung mit einer Menge Alkylalkohol oder Wasser entsprechend 0,2 bis 10, insbesondere 0,5 bis 4, bevorzugt mit 1 bis 4 Mol je Mol Benzylchlorid der Formel (II) durch.

Beim Einsatz Benzylchloriden der Formel (II), worin mindestens einer der Reste R¹, R², R³, R⁴, und R⁵ unabhängig voneinander einen CH₂Cl-Rest darstellt, erhält man entsprechend dem erfindungsgemäßen Verfahren Phenylessigsäuren der Formel (I), worin mindestens einer der Reste R¹, R², R³, R⁴, und R⁵ unabhängig voneinander einen CH₂Cl-Rest darstellt. Diese Produkte sind entsprechend dem beschriebenen Verfahren wieder Benzylchloride der Formel (II) und werden entsprechend dem beschriebenen Verfahren weiter umgesetzt. Folglich entspricht ein Mol Benzylchlorid der Formel (II), worin mindestens einer der Reste R¹, R², R³, R⁴, und R⁵ unabhängig voneinander einen CH₂Cl-Rest darstellt, der Zahl der CH₂Cl-Gruppen entsprechend viele Mole Benzylchlorid.

Beim Einsatz Benzylchloriden der Formel (II), worin mindestens einer der Reste R¹, R², R³, R⁴, und R⁵ unabhängig voneinander einen CH₂Cl-Rest darstellt und worin mindestens zwei CH₂Cl-Gruppen ortho-ständig sind, erhält man neben den Phenylessigsäuren der Formel (I) auch 3-Isochromanon-Derivate der allgemeinen Formel (III).

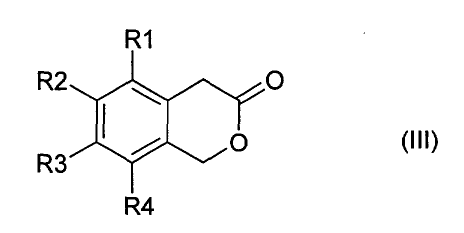

Nach einer besonderen Ausführungsform des erfindungsgemäßen Verfahrens legt man das Benzylchlorid der Formel (II), den Katalysator, das dipolar aprotische Lösemittel und gegebenenfalls auch die Verbindung R⁶OH sowie gegebenenfalls das ionische Halogenid vor, stellt den CO-Druck und die Temperatur ein und dosiert anschließend den Alkylalkohol oder das Wasser oder ein aus dem Alkylalkohol oder Wasser und dem dipolar aprotischen Lösungsmittel bestehendes Gemisch zu.

Während der Umsetzung sorgt man für eine gute Durchmischung der Reaktionspartner, um einen zügigen Reaktionsverlauf zu gewährleisten.

Das erfindungsgemäße Verfahren eignet sich sowohl für eine kontinuierliche als auch diskontinuierliche Durchführung.

Die nachfolgenden Beispiele beschreiben die Erfindung, ohne sie darauf zu beschränken.

### Beispiele:

### Herstellung von Phenylessigsäure

### Beispiel 1

In einem 300 ml HC4-Autoklav wird zu einer Lösung von 36,9 g Benzylchlorid [291,5mmol] und 26 mg Palladiumchlorid [0,145mmol] in 76 g N-Methylpyrrolidon bei einem Druck von 30 bar Kohlenmonoxid und einer Temperatur von 150°C, 11,6 g einer Lösung aus 50 Gew.-% Wasser [321mmol] in N-Methylpyrrolidon über einen Zeitraum von 1 Stunden eindosiert. Der Druck wird während der Zudosierung konstant auf 30 bar gehalten. Nach ca. 10 Stunden Nachrührzeit ist die Kohlenmonoxidaufnahme beendet. Nach dem Abkühlen erhält man eine GC-Ausbeute von 87 % an Phenylessigsäure. Nach fraktionierter Destillation erhält man 32,1 g Phenylessigsäure [278,7mmol], entsprechend einer praktischen Ausbeute von 81 %. Der Schmelzpunkt beträgt 78°C.
Die Fraktion der Leichtsieder enthält N-Methylpyrrolidon und nicht umgesetztes Benzylchlorid.

### Beispiel 2

In einem 300 ml HC4-Autoklav wird zu einer Lösung von 35 g o-Methylbenzylchlorid [248,9mmol] und 22 mg Palladiumchlorid [0,124mmol] in 75 g N-Methylpyrrolidon bei einem Druck von 20 bar Kohlenmonoxid und einer Temperatur von 130°C, 10 g einer Lösung aus 4,9 g Wasser [273,8mmol] in 5 g N-Methylpyrrolidon über einen Zeitraum von 1 Stunde eindosiert. Der Druck wird während der Zudosierung konstant auf 20 bar gehalten Nach Beendigung der Zudosierung wird 12 Stunden, bis zur Druckkonstanz weiter erhitzt. Die Aufarbeitung erfolgt analog Beispiel 1.

Die praktische Ausbeute beträgt nach Destillation 79 % an o-Methylphenylessigsäure [197,5mmol]. Der Schmelzpunkt liegt bei 88-90°C, die GC-Reinheit beträgt 100 %.

### Beispiel 3

In einem 300 ml HC4-Autoklav wird zu einer Lösung von 35 g o-Chlorbenzylchlorid [217,4 mmol] und 20 mg Palladiumchlorid [0,109mmol] in 75 g N-Methylpyrrolidon, bei einem Druck von 20 bar Kohlenmonoxid und einer Temperatur von 130°C eine Lösung aus 4,3 g Wasser [239mmol] in 5 g N-Methylpyrrolidon über einen Zeitraum von 1 Stunde eindosiert. Der Druck wird während der Zudosierung konstant auf 20 bar gehalten.
Nach Beendigung der Zudosierung wird 9 Stunden bis zur Druckkonstanz weiter erhitzt. Die Aufarbeitung erfolgt nach dem Abkühlen analog Beispiel 1.
Die praktische Ausbeute beträgt 82 %, das entspricht einer Masse von 30,3 g an o-Chlorphenylessigsäure mit einer GC-Reinheit von 100 % und einem Schmelzpunkt von 95-97°C.

### Beispiel 4

In einem 300 ml HC4-Autoklav wird zu einer Lösung von 49 g 2,4-Dichlorbenzylchlorid [252,5 mmol] und 18 mg Palladiumchlorid [0,1 mmol] in 105 g N-Methylpyrrolidon, bei einem Druck von 20 bar Kohlenmonoxid und einer Temperatur von 130°C, 10 g einer Lösung aus 5 g Wasser [278 mmol] und 5 g N-Methylpyrrolidon über einen Zeitraum von 1 Stunde eindosiert. Der Druck wird während der Zudosierung konstant auf 20 bar gehalten. Es wird bis zur Druckkonstanz, die nach 16 Stunden erreicht wird, nachgerührt. Nach dem Abkühlen wird das N-Methylpyrrolidon sowie der Chlorwasserstoff bei 150°C und 200 mbar abgetrennt. Der Rückstand wird als Schmelze in Wasser unter paralleler Eindosierung von 50 %iger wässeriger Natriumhydroxydlösung, so dass der pH-Wert 10 gehalten wird. Man erhält eine homogene Lösung, die mit 20 ml Xylol extrahiert wird um organisch lösliche Stoffe abzutrennen.
Anschließend wird mit ca. 30 %iger wässeriger Salzsäure auf pH 1 angesäuert, wobei die 2,4-Dichlorphenylessigsäure als weißer Feststoff ausfällt. Das ausgefallene Produkt wird mit soviel Wasser gewaschen, bis das ablaufende Abwasser einen konstanten pH-Wert hat. Das gewaschene Produkt wird bei 100°C und erniedrigten Druck getrocknet.
Die praktische Ausbeute beträgt 89 % mit einer GC-Reinheit von 100 % an 2,4-Dichlorpenylessigsäure. Der Schmelzpunkt beträgt 131-133°C.

## Patentansprüche

1. Verfahren zur Herstellung von Phenylessigsäurederivaten der allgemeinen Formel (I) durch Umsetzung von Benzylchloriden der allgemeinen Formel (II) 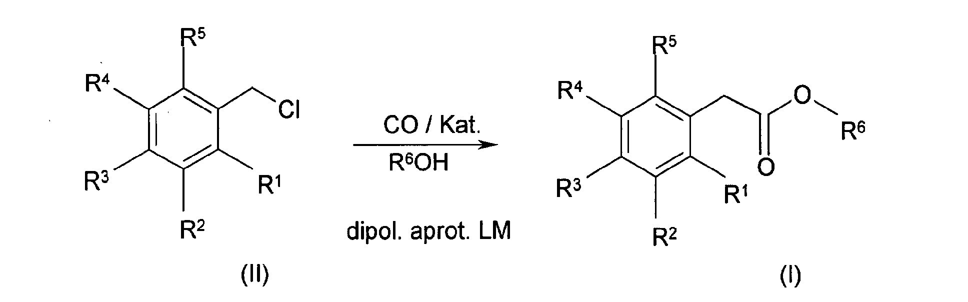 mit einer Verbindung der allgemeinen Formel R⁶OH und mit Kohlenmonoxid in einem dipolar aprotischen Lösungsmittel in Gegenwart eines Katalysators, der mindestens eine Verbindung eines Übergangsmetalls der VIII. Nebengruppe des Periodensystems enthält,
wobei R¹, R², R³, R⁴, und R⁵ unabhängig voneinander für folgende Reste stehen:
ein Wasserstoff oder Fluoratom;
ein CH₂Cl-Rest;
eine HO₂CCH=CH-, NC- oder CF₃-Gruppe;
einen Alkyl-, Alkoxy-, oder Acyloxyrest, mit jeweils 1 bis 18 Kohlenstoffatomen; oder einen C₆-C₁₈-Aryloxy, Aryl- oder Heteroarylrest, wobei als Heteroatome 1 bis 3 Atome der Gruppe O, N und/oder S vorhanden sind;
einen R⁸₂P(=O)-, R⁹C(=O)-, R⁹OC(=O)-, R⁹OC(=O)CH=CH-, R¹⁰C(=O)-,
R¹⁰OC(=O)-, R¹⁰OC(=O)CH=CH-, oder R¹⁰₂P(=O)-Rest, worin R⁸ einen C₁-C₄-Alkylrest, R⁹ einen C₁-C₁₈-Alkylrest oder Wasserstoff und R¹⁰ einen C₆-C₁₈-Arylrest darstellen;
oder worin mindestens zwei der Reste R¹, R², R³, R⁴, und R⁵ untereinander verknüpft sind und mindestens einen aliphatischen oder aromatischen Ring mit 5 bis 18 C-Atomen bilden;
R⁶ steht für Wasserstoff oder einen C₁-C₁₈-Alkyl- oder C₆-C₁₈-Arylrest.

2. Verfahren nach Anspruch 1, **dadurch gekennzeichnet, dass** R¹, R², R³, R⁴, und R⁵ unabhängig voneinander Wasserstoff, Fluor, einen C₁-C₈-Alkyl- oder einen C₁-C₈-Alkoxyrest bedeuten oder zwei der Reste R¹, R², R³, R⁴, und R⁵ untereinander verknüpft sind und einen aliphatischen oder aromatischen Ring mit 5 bis 10 C-Atomen bilden.

3. Verfahren nach Anspruch 1 oder 2, **dadurch gekennzeichnet, dass** R⁶ für einen C₁-C₄-Alkylrest oder Wasserstoff steht.

4. Verfahren nach einem oder mehreren der vorigen Ansprüche, **dadurch gekennzeichnet, dass** die Umsetzung in Gegenwart eines ionischen Halogenids durchgeführt wird.

5. Verfahren nach Anspruch 4, **dadurch gekennzeichnet, dass** als ionisches Halogenid ein Alkali- oder Ammoniumhalogenid oder ein Alkylammoniumhalogenid oder ein Phosphoniumhalogenid verwendet wird.

6. Verfahren nach mindestens einem der vorhergehenden Ansprüche, **dadurch gekennzeichnet, dass** der Katalysator Palladium-, Nickel- und/oder Kobaltverbindungen enthält.

7. Verfahren nach Anspruch 6, **dadurch gekennzeichnet, dass** der Katalysator zusätzlich einen Liganden enthält.

8. Verfahren nach Anspruch 7, **dadurch gekennzeichnet, dass** der Ligand eine Phosphinverbindung ist.

9. Verfahren nach mindestens einem der vorhergehenden Ansprüche, **dadurch gekennzeichnet, dass** der Katalysator in einer Menge von 0,00001 bis 0,3 Mol Übergangsmetall je Mol Benzylchlorid der Formel (II) eingesetzt wird.

10. Verfahren nach mindestens einem der vorhergehenden Ansprüche, **dadurch gekennzeichnet, dass** die Umsetzung bei einem CO-Druck von 0,5 bis 20 MPa durchgeführt wird.

11. Verfahren nach mindestens einem der vorhergehenden Ansprüche, **dadurch gekennzeichnet, dass** die Umsetzung bei einer Temperatur im Bereich von 20 bis 220°C durchgeführt wird.
